Europäisches Patentamt

(19) European Patent Office

Office européen des brevets

(11) Publication number: **0 218 275**
**A1**

## EUROPEAN PATENT APPLICATION

(21) Application number: **86201487.5**

(22) Date of filing: **29.08.86**

(51) Int. Cl.⁴: **A 61 M 25/00**

(30) Priority: **30.08.85 NL 8502382**

(43) Date of publication of application: **15.04.87**
**Bulletin 87/16**

(84) Designated Contracting States: **DE FR GB IT NL**

(71) Applicant: **Fijneman, Martinus Jacobus Antonius Johannes, Willem de Zwijgerlaan 54, NL-2082 BD Santpoort (NL)**

(72) Inventor: **Neligan, Maurice Christopher, Den Inagh House 2, Rockroad, Blackrock, C.O. Dublin (IE)**

(74) Representative: **Smulders, Theodorus A.H.J. et al, Vereenigde Octrooibureaux Nieuwe Parklaan 107, NL-2587 BP 's-Gravenhage (NL)**

(54) **Multi-purpose catheter.**

(57) A catheter having the form of a hollow tube (6) comprising near its end destined to be inserted into a human or animal body a balloon (8) which is inflatable from the other end of the tube (6) through a lumen in the tube, said hollow tube comprising at least one other lumen for fluid transport through the catheter tube, for the manufacture of a surgery expedient for use in cardiovascular surgery to direct the blood stream to by-pass the heart (1) through an extracorporeal trajectory (15).

Title: Multi-purpose catheter.

The invention relates to a catheter in the form of a hollow tube comprising near its end destined to be inserted into the body, a balloon which through a passage in the tube is inflatable from the other end of the tube and in which further at least one lumen extends for the transport of fluids through the catheter tube.

Such catheters are known per se for application in a urinary duct wherein by inflation of the balloon the urine passage is closed off and urine can be drained via the catheter. Such catheters are further used as endotracheal tubes and catheters with an inflatable balloon are also used to scrape accumulated matter from the inner wall of arteries.

The object of the invention is to provide such a catheter which can be applied as an expedient during open-heart surgery.

During operations on the heart the heart has to be arrested and must be dry. Therefore that section of the blood circuit which goes through the heart is isolated and the blood stream is diverted to by-pass the heart via an extracorporeal trajectory comprising at least a heart-lung machine and an oxygenator. Deoxygenated blood is drained from the body and oxygenated blood is redelivered to the body. Further blood vessels and connections between heart and lungs have to be closed off. Also, blood remaining in the isolated area has to be removed and the drainage means has to remain in its place during the operation to drain off any fluid which finds its way into the isolated area during the operation. The closing of the vessels is done with clamps whose size and type depend on the blood vessel to which the clamp is to be applied.

During open-heart surgery a cold crystalloid liquid, so-called cardioplegic, is introduced into the heart muscle via the coronary arteries. This cold liquid causes complete cessation of all electric activity of the heart and the heart muscle no longer gets oxygen. Should the heart muscle use oxygen without the supply of fresh oxygen, the heart muscle could be damaged seriously.

Therefore during a heart operation at least the following expedients are necessary.

- A catheter to drain deoxygenated blood from the right atrium and from the vein which transports this blood to the heart and to supply this to the extracorporeal trajectory.

- A cannula to supply oxygenated blood from the extracorporeal trajectory to the aorta.

- A catheter to drain the left atrium during the diversion of the blood to by-pass the heart and lungs.

- A cannula to serve as a conduit to deliver the cold cardioplegic liquid to the heart.

- Finally, clamps to be placed at desired sites on blood vessels to occlude these entirely or partially.

For all afore mentioned catheters and cannulae separate incisions have to be made and some types of catheters require even more than one, for example for the two blood vessels which supply venous blood to the heart. The size of each incision depends on the sizes of the cannulae or catheters to be inserted at that site.

The invention provides a catheter which is applicable for all the afore-mentioned functions during open-heart surgery and which is characterized in that the catheter tube contains, apart from a lumen for inflating the balloon, which lumen can also be situated at the outside of the catheter, at least two internal lumina for the delivery and drainage of fluids to and from the zone near the balloon with which a blood vessel can be occluded.

Relative to a conventional catheter applied during heart operations a balloon catheter has the advantage that it requires a smaller incision because the balloon is inflated only after arrival at the desired site within the blood vessel. Further, occlusion of a blood vessel with the help of a balloon causes less trauma to a blood vessel or a heart muscle than a conventional clamp because a balloon is more flexible. By using a balloon catheter the position of the tip of the catheter is better controlled than in the case of a catheter which is inserted through a vascular wall and is fixed with a clamp. The balloon in fact keeps the tip of the catheter spaced from the vessel wall so the blood vessel tissue cannot obstruct the flow through the tip of the catheter in any way. Further the conventional clamps for occluding blood vessels form obstacles in the operation zone which reduce accessibility of that area. By using a balloon catheter as a vessel occlusion means this drawback is avoided.

In one embodiment of the present invention the catheter tube comprises three separate channels extending from respective connection nipples at the rear end, to the interior of the balloon, to the tip of the catheter behind the balloon and to one or more apertures in the wall of the catheter behind the balloon, respectively. Such catheters can be used for insertion into the aorta, near the aortic valve, at the entrance thereof at the heart side and also in the or each vena cava. After inflation of the respective balloons the extracorporeal circuit over the heart-lung machine can be closed via a blood transport lumen which extends through the catheter, while via the at least one further, third, channel present in both types of catheters cardioplegic fluid can be delivered between the balloon situated in the aorta and the heart so that it reaches the coronary arteries. After arresting the heart function the cardioplegic solution may be removed through the same lumen as well as via the said third channel in the catheters positioned in the blood vessels.

The balloons should preferably be designed as solids of revolution with generatrices in the form of ellipses with their longer axis at right angles to the central axis of the catheter, while the surfaces of the balloons may be provided with ribs extending circumferentially over the balloon surfaces transversely to the central axis of the catheter, which ribs ensure a firm grip on the inner wall of a blood vessel without too much pressure being needed.

The form and flexibility of the tip of the catheter will preferably be selected to suit the specific application of the catheter.

A rounded tip having lateral inlet holes ensures maximum drainage from the veins which deliver deoxygenated blood to the heart.

For other applications the free tip of the catheter can be angled for enabling suction of small quantities of fluid collecting in the bottom region of blood vessel. The balloon ensures that the tip of the catheter is kept spaced from the blood vessel wall. In such a case the catheter should have a line marker so that the direction of the angled tip may be observed from the outside.

For a better understanding of the invention some embodiments of the multi-purpose catheter will be described, by way of example, with reference to the accompanying drawings.

Fig. 1 schematically shows two applications of the catheter; and

Fig. 2 shows, also schematically, a third application of the catheter.

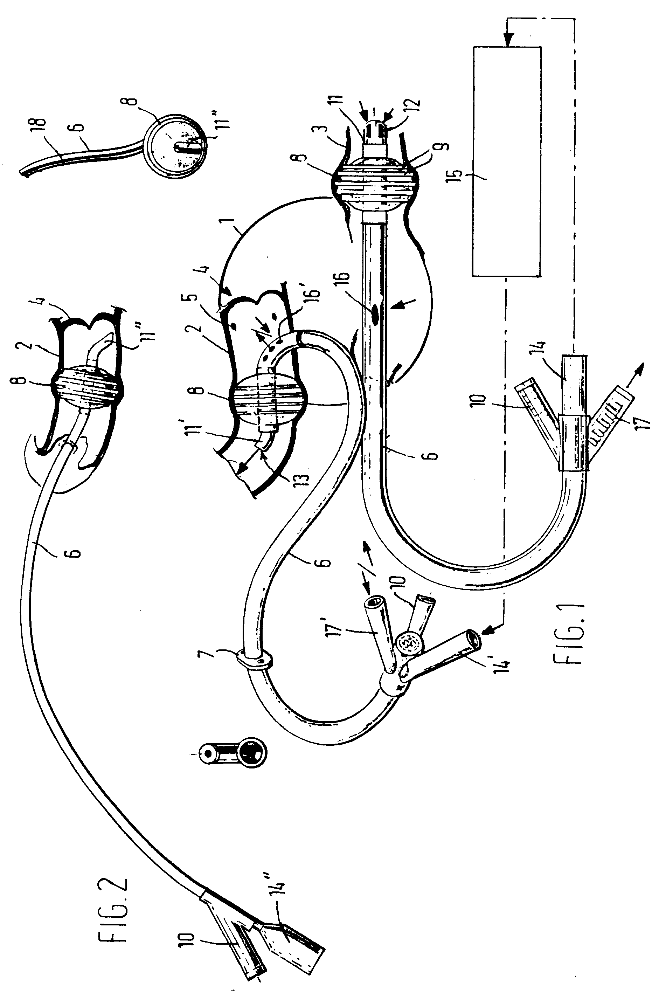

Fig. 1 shows the heart schematically as a circle 1 as well as aorta 2 and a blood vessel 3 that normally delivers deoxygenated blood to the heart. At the entrance of the aorta the aortic valve 4 is shown with directly downstream thereof, inlets 5 of the coronary artery (not shown). Neither the lungs or their connection with the heart are shown. Normal blood circulation is from the veins 3 to the heart 1, from the heart to the lungs and back again to the heart, and via the valve 4 into the aorta 2 through which the blood is distributed via the arteries through the body, and via veins is returned to blood vessels 3.

To perform an operation on the heart the blood circulation should be led to by-pass the heart and the heart functions must be arrested, the heart should not contain any fluid.

For this purpose catheters, according to the invention, are introduced into the veins 3 as well as into the aorta 2. Each of these catheters, shown in Fig. 1 comprise a catheter tube 6 which at the outside can be provided with one or more attachment elements 7. Provided near the free end of each catheter tube 6 is a balloon 8, which has transverse ribs 9. The ballons 8 are inflatable via a lumen extending through the catheter tube 6 and at the rear end of the catheter tube having a connector 10. Further every catheter is furnished at the free end with a tip 11, 11', respectively, which is designed to suit its specific application. The tip 11 is provided with lateral inlet openings 12 and the tip 11' has a central opening 13. The openings 12, 13 communicate with another lumen also extending through the catheter tube 6 and having its own connector nipples 14 or 14'. The nipples 14 and 14' are connected to a heart-lung machine 15 in a manner shown in Fig. 1.

The catheters shown in Fig. 1 are further provided with a third lumen extending through the catheter tube 6 and connecting apertures 16 or 16' in the catheter tube with its own connector 17 or 17'. This third lumen serves for transport of cardioplegic and other fluids.

The function of the catheters in the situation shown schematically in Fig. 1 is as follows.

After positioning the catheters as indicated the respective balloons 8 are inflated via the connectors 10 in order to close off the blood vessel 3 and the aorta 2. The connecting nipples 14 and 14' are connected as indicated with the heart-lung machine, or in general with the extracorporeal blood circuit. After draining the heart, which will be described in more detail hereinafter, cardioplegic fluid is delivered to the heart via nipple 17'. This liquid, flowing out at opening 16' reaches the coronary artery via the opening 5 and cools it to the extent that the heart ceases to function. After this the cardioplegic fluid is withdrawn again via the same openings 16' and the connecting nipple 17' and also via the opening 16 in the other catheter and the corresponding connecting nipple 17. All this is indicated by arrows in the drawing. The heart now is ready for the operation and the blood circulates via the openings 12, the connecting nipple 14, the heart-lung machine 15, the nipple 14' and the opening 13 in the catheter tip 11'.

To keep the heart dry during the operation the accumulated fluid can be sucked away via openings 16 and 16'. Further the same type of catheters can be used in various areas of the heart, if necessary with an adapted form of the tip of the catheter, as shown in Fig. 2. In this figure like parts are designated by the same reference numerals as in Fig. 1.

In Fig. 2, the catheter is positioned in the aorta, but reversed relative to the position shown in Fig. 1. The tip 11' is curved downwards so that even small quantities of fluid remaining in the aortic root can be drained away. To ensure that the tip 11' is curved downwards a marker line 18 is positioned on the catheter tube 6.

A catheter as shown in Fig. 2 can also be placed in a ventricle, in which case the balloon 8 does not serve to occlude any blood vessel but only to prevent that after introduction of the catheter in the heart and inflation of the balloon 8, the catheter is dislodged from the heart.

The lumen between connection 14" and the tip 11" can also be used as a temporary means for supplying cardioplegic solution. In the application of the catheter shown in Fig. 2 it is sufficient to use only two channels in the catheter tube 6, namely one for inflating the balloon 8 and the other for delivery of cardioplegic fluid and for draining the zone beyond balloon 8.

Making use of the presence of a plurality of lumina in the catheter tube it is possible, if so desired, for example for facilitating the insertion of an extremely flexible catheter tube, and/or when inserting a catheter moving oppositely to the blood stream, to apply a known per se pilot wire. Such a thin flexible pilot wire is pushed into the blood vessel with its leading end a head of the catheter tip and the catheter, with the pilot wire extending through one of its lumina, is pushed in over the pilot wire thus inserted.

-10-

## CLAIMS

1. The use of a catheter having the form of a hollow tube (6) comprising near its end destined to be inserted into a human or animal body a balloon (8) which is inflatable from the other end of the tube (6) through a lumen in the tube, said hollow tube comprising at least one other lumen for fluid transport through the catheter tube, for the manufacture of a surgery expedient for use in cardiovascular surgery to direct the blood stream to by-pass the heart (1) through an extracorporeal trajectory (15).

2. The use of a catheter having the form of a hollow tube (6) comprising near its end destined to be inserted into a human or animal body a balloon (8) which is inflatable from the other end of the tube (6) through a lumen in the tube, said hollow tube comprising at least one other lumen for fluid transport through the catheter tube, for the manufacture of a surgery expedient for use in cardiovascular surgery to occlude the vena cava (3) and to drain the blood therefrom and pass it to a heart-lung machine (15).

3. The use of a catheter having the form of a hollow tube (6) comprising near its end destined to be inserted into a human or animal body a balloon (8) which is inflatable from the other end of the tube (6) through a lumen in the tube, said hollow tube comprising at least one other lumen for fluid transport through the catheter tube, for the manufacture of a surgery expedient for use in cardiovascular surgery, to occlude the aorta (2) and to pass blood from a heart-lung machine (15) to the aorta (2).

4.    The use of a catheter having the form of a hollow tube (6) of a size larger than about 10.7 mm comprising a rounded end (11) with inlet holes (12) to be inserted into a human body and to be positioned into the vena cava (3), near said inlet holes (12) a balloon (8) which is inflatable from the other end (10) of the tube (6) through a lumen in the tube, and at least one other lumen for the transport of blood through the catheter tube (6), for the manufacture of a surgery expedient for use in cardiovascular surgery to direct the blood stream from the vena cava to by-pass the heart (1) to an extracorporeal trajectory (15).

5.    The use of a catheter having the form of a hollow tube (6) comprising a lumen in the tube (6) for transport of fluid and having at its end (11') destined to be inserted into a human body and to be positioned into the aorta (2) an axially facing opening (13) communicating with said lumen, a balloon (8) near said opening (13) inflatable from the other end (10) of said tube (6) through a further lumen in said tube, for the manufacture of a surgery expedient for use in cardiovascular surgery to direct blood from a heart-lung machine (15) into the aorta (2).

6.    The use of a catheter having the form of a hollow tube (6) comprising a balloon and three lumina, said balloon being attached to the tube near its end destined to be inserted into either the vena cava or the aorta of a human or a warm-blooded animal and being inflatable from the other end of the tube (6) through one of said lumina in the tube, a second lumen in the tube being provided with at least one opening in the terminal part of the tube located beyond the balloon for transport of blood through the catheter tube, and a third lumen in the tube being provided with at least one opening located in front of the balloon for transport of cardioplegic liquid through the catheter tube, for the manufacture of a surgery expedient for use in cardiovascular surgery to direct the blood stream to by-pass the heart (1) through an extracorporeal trajectory (15) and to deliver cardioplegic liquid to the heart.

7. A catheter for directing the blood circulation in a human body to by-pass the heart (1) through an extracorporeal circuit, comprising a hollow catheter tube (6) having near its end (11, 11') destined to be inserted into the human body, a balloon (8) inflatable from the other end of the tube through a lumen in the tube (6), to occlude the vena cava (3) or the aorta (2) and having at least one other lumen in said tube (6) for draining blood from the vena cava (3) into the said extracorporeal circuit (15) and to introduce blood from said extracorporeal circuit into the aorta (2) respectively.

8. A catheter according to claim 7, characterized by a third lumen in the tube (6) extending from a connector (17, 17') to an aperture (16, 16') at the proximal side of the balloon (8).

9.    A catheter according to claim 7 or 8, characterized in that for use to occlude and drain the vena cava, the tube (6) has a size of at least about 10.7 mm.

10.    A catheter according to claim 7 or 8, characterized in that for use to occlude and feed the aorta (2) the distal end (11') of the tube (6) has an axially-oriented opening (13).

FIG.1

FIG.2

European Patent Office

**PARTIAL EUROPEAN SEARCH REPORT**
which under Rule 45 of the European Patent Convention shall be considered, for the purposes of subsequent proceedings, as the European search report

**0 218 275**

EP  86 20 1487

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 148 131 (U. PASQUI et al.)<br>* Figures 1-4; abstract * | 7,10 | A 61 M 25/00 |
| Y | | 8 | |
| Y | NL-A-77 07 741 (HOECHST AG)<br>* Figure 1; claims 1-4 * | 8 | |
| A | US-A-4 154 227 (KRAUSE et al.)<br>* Figures 1,3; column 1, line 67 - column 2, line 6 * | 10 | |
| A | EP-A-0 150 960 (CEDARS-SINAI MED. CENTER)<br>* Figure 4; page 9, lines 24-28 * | 7,10 | |
| A | EP-A-0 080 436 (SCHNEIDER MEDINTAG)<br>* Figures 1,7,9; abstract * | 7,8,10 | **TECHNICAL FIELDS SEARCHED (Int. Cl.4)**<br>A 61 M |

### INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely:  7-10 (Catheter)

Claims searched incompletely:

Claims not searched:  1-6 (Use of catheter)

Reason for the limitation of the search:

Method for treatment of the human or animal body by surgery or therapy (see art. 52(4) of the European Patent Convention).

| Place of search | Date of completion of the search | Examiner |
|---|---|---|
| The Hague | 12-12-1986 | JONES |

EPO Form 1505.1 03.82